# FASCICULE DE BREVET EUROPEEN

(11) **EP 0 536 066 B1**
(45) Date de publication et mention de la délivrance du brevet: **17.04.1996**
(21) Numéro de dépôt: 92420340.9
(22) Date de dépôt: 29.09.1992
(51) Int. Cl.: A61B 17/58, A61B 17/70

(54) **Dispositif d'ostéosynthèse vertébrale**
Osteosynthesevorrichtung für die Wirbelsäule
Vertebral osteosynthesis device

(30) Priorité: 30.09.1991 FR 9112206
(43) Date de publication de la demande: 07.04.1993
(73) Titulaire: FIXANO SA, F-01000 Bourg en Bresse (FR)
(72) Inventeur: Martin, Jean-Jacques, F-01000 Bourg en Bresse (FR); Lescuyer, Jean-Philippe, F-01000 Bourg en Bresse (FR); Cartoux, René, F-84220 Gordes (FR)
(74) Mandataire: Maureau, Philippe

(56) Documents cités:
- EP-A- 0 346 521
- EP-A- 0 383 992
- WO-A-91/01691
- DE-B- 2 345 363

## Description

La présente invention concerne un dispositif d'ostéosynthèse vertébrale, pouvant être utilisé pour simplement étayer un rachis qui en a besoin, par exemple à la suite d'une fracture accidentelle, ou pour redresser et étayer un rachis objet de déviations, telles que scoliose ou cyphose.

Un tel dispositif comprend fréquemment et de manière bien connue en soi, deux tiges rigides de support sensiblement parallèles disposées chacune d'un côté des apophyses épineuses des vertèbres et sur lesquelles peuvent être montés des éléments d'ancrage osseux, constitués soit par des crochets destinés à être engagés autour des apophyses vertébrales, soit par des vis destinées à prendre appui dans le pédicule des vertèbres.

L'implantation d'un dispositif de ce type rend nécesaire la mise en forme précise des tiges pour les adapter aux courbures dans un ou deux plans du rachis, qu'il s'agisse de courbures naturelles ou résultant de cyphoses ou de scolioses et implique l'obtention d'une parfaite liaison entre les tiges et les éléments d'ancrage, cette liaison devant être capable de résister dans le temps aux contraintes répétées auxquelles sont soumis les différents éléments par les mouvements du patient.

Les demandes de brevets internationale N° WO 91/01691 et européen N° 0 346 521 décrivent plus spécifiquement des éléments d' ancrage comprenant, d'une part, deux parois latérales faisant saillie du côté opposé à leur organe d'accrochage, sensiblement parallèles et flexibles, délimitant entre elles un canal de largeur sensiblement égale au diamètre des tiges et, d'autre part, un élément de serrage apte à être engagé sur les parois latérales et à les rapprocher l'une de l'autre au cours dudit engagement. Les tiges sont introduites dans les canaux puis les éléments de serrage sont mis en place. Le serrage radial exercé sur la tige par la déformation desdites parois et l'importante surface de contact entre la tige et les parois assurent la solidarisation des éléments d'accrochage à la tige.

La mise en forme précitée des tiges n'est guère aisée avec ces dispositifs, puisqu'il est difficile, alors que la tige n'est pas maintenue entre les pattes flexibles, de vérifier si la forme donnée aux tiges convient ou non, ou si cette forme doit être retravaillée. De plus, la résistance dans le temps de la liaison éléments d'ancrage/tiges est incertaine. compte tenu de ce que la fixation des éléments de serrage dépend de la flexion des pattes.

Les éléments de serrage présentent, en outre, les inconvénients d'être difficiles à mettre en place et à serrer, et de ne pas permettre au praticien de parfaitement contrôler le serrage de la tige qu'ils produisent. En effet, le serrage est rendu difficile par la déformation des parois. De plus, l'accès aux éléments de serrage est plutôt limité et est problématique quand les éléments de serrage sont constitués par des écrous pouvant être vissés sur l'extrémité des parois : le débattement possible de la clé de serrage est en effet particulièrement faible compte tenu des dimensions de l'incision et de la présence des différents instruments.

Par ailleurs, il peut être intéressant dans certains cas de réaliser un serrage juste suffisant pour immobiliser temporairement les tiges par rapport aux éléments d'accrochage mais sans les bloquer définitivement par rapport à eux, notamment pour une ultime vérification du réglage de la courbure des tiges.

La présente invention vise à fournir un dispositif d'ostéosynthèse vertébrale qui remédie à l'ensemble de ces inconvénients.

A cette fin, dans le dispositif qu'elle concerne, la distance séparant les faces intérieures des parois latérales, à proximité de leur extrémité supérieure, est inférieure au diamètre des tiges, afin que ces dernières puissent être maintenues provisoirement entre elles par clipsage, et les faces extérieures des parois latérales sont inclinées l'une en direction de l'autre de manière à converger du côté opposé à l'organe d'accrochage destiné à être fixé à l'os, l'élément de serrage ayant une forme d'étrier, dont les branches latérales possèdent des faces intérieures inclinées selon des pentes qui correspondent à celles des faces extérieures des parois latérales, et comprenant au moins deux moyens de serrage, disposés au-delà des parois latérales, prévus pour déplacer ledit élément de serrage de manière à ce que les faces intérieures de ses branches latérales viennent appuyer contre les parois latérales en les rapprochant l'une de l'autre et glisser sur elles lors du serrage.

L'invention permet ainsi une mise en place provisoire et un retrait aisé des tiges hors des éléments d'ancrage, pour permettre d'apprécier si la forme donnée aux tiges convient ou non et de la retravailler, si besoin est.

De plus, la liaison procurée par l'invention est tout-à-fait solide et résistante dans le temps puisque les moyens de serrage de l'étrier sur la tête des vis ou des crochets ne prennent pas appui sur les pattes latérales flexibles. Ces dernières, une fois l'étrier serré, sont parfaitement soutenues et peuvent dès lors avoir la flexibilité relative nécessaire pour permettre le clipsage précité.

Le fait de prévoir plusieurs moyens de serrage disposés latéralement permet, en outre, de parfaitement contrôler la déformation des parois latérales et, donc, le serrage exercé par elles sur la tige. La précision dans le serrage ainsi obtenue permet dès lors de réaliser un serrage juste suffisant pour immobiliser la tige par rapport aux éléments d'accrochage sans la bloquer définitivement, pour une ultime vérification de l'adéquation de sa forme aux courbures du rachis.

Globalement, l'invention permet une réduction très notable de la durée de l'opération chirurgicale et, par conséquent, de réduire la durée de l'anesthésie que doit subir le patient et la consommation en produits sanguins perfusés.

Avantageusement, lesdits moyens de serrage sont constitués par deux vis, dont les têtes sont en appui contre la face extérieure de la partie centrale de l'étrier et dont les corps traversent les branches parallèles de celui-ci, ces vis étant engagées dans des alésages taraudés ménagés dans deux saillies situées à l'extérieur des parois latérales, que comprend chaque élément d'accrochage.

Les vis sont ainsi parfaitement accessibles et faciles à manipuler, en particulier lorsque leurs têtes sont du type hexagonal.

Selon une possibilité, la partie centrale des parois latérales est usinée, voire évidée, pour permettre le passage des vis. Un gain d' encombrement sensible des éléments d'accrochage est ainsi obtenu.

De préférence, deux fentes sont ménagées dans chaque élément d'accrochage, entre les parois latérales et à proximité de leur base. Ces fentes confèrent une flexibilité accrue aux parois latérales, facilitant le serrage. Selon une forme de réalisation préférée de l'invention, ces fentes sont ménagées radialement par rapport à la tige.

De toute façon, l'invention sera bien comprise à l'aide de la description qui suit, en référence au dessin schématique annexé, représentant, à titre d'exemple non limitatif, une forme de réalisation préférée du dispositif d'ostéosynthèse vertébrale qu'elle concerne.
Figure 1 en est une vue générale, alors qu'il est monté sur un rachis ;
Figures 2 et 3 en sont des vues partielles, respectivement en perspective éclatée et en perspective montée ; et
Figure 4 en est une vue en coupe selon IV-IV de figure 1.

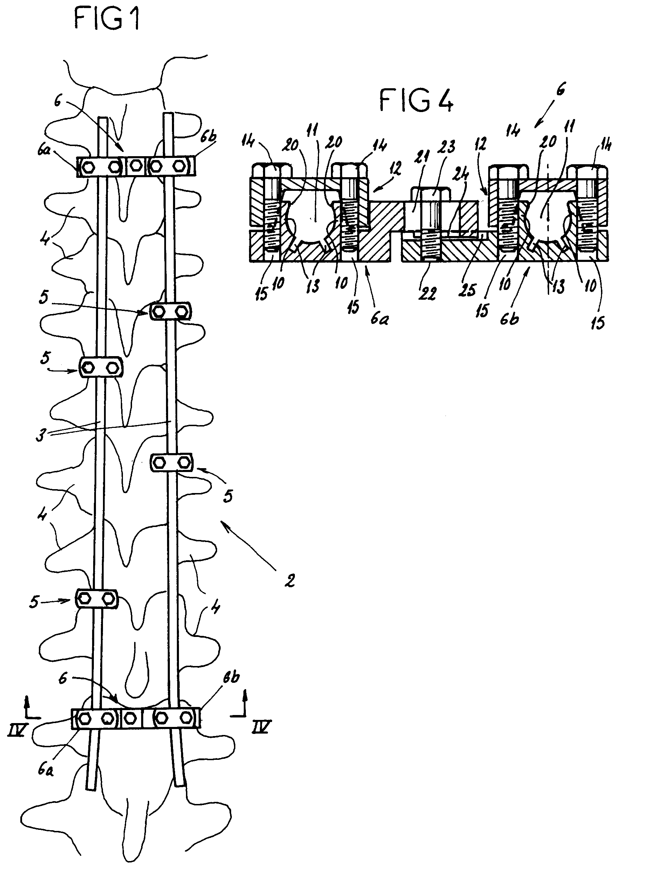

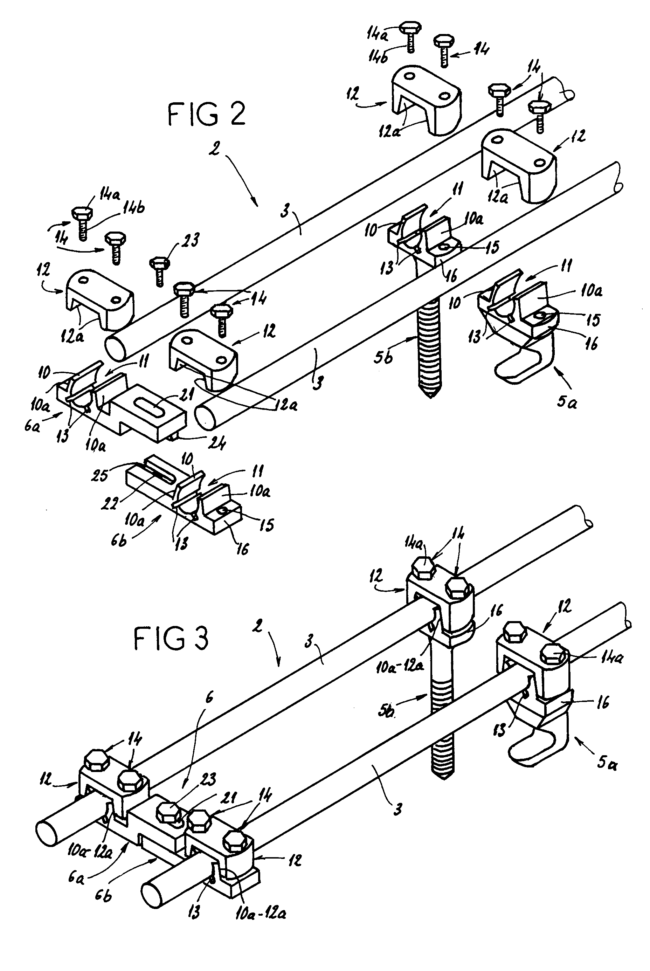

La figure 1 représente un dispositif d'ostéosynthèse vertébrale 2 comprenant deux tiges rigides de support 3, sensiblement parallèles et disposées chacune d'un côté des apophyses épineuses des vertèbres 4. Sur les tiges 3 peuvent être montés des éléments 5 d'ancrage osseux, pouvant être constitués, ainsi que le montrent plus particulièrement les figures 2 et 3, soit par des crochets 5a destinés à être engagés autour des apophyses vertébrales, soit par des vis 5b destinées à prendre appui dans le pédicule des vertèbres 4. Le dispositif 2 comprend également au moins un élément transversal 6, constitué de deux pièces 6a et 6b assemblées l'une à l'autre, ainsi que le montrent les figures 2 à 4. Les éléments 6 sont destinés à relier les tiges 3 l'une à l'autre.

Ainsi que cela apparaît sur les figures 2 à 4, chacun des éléments d'accrochage 5 ou des pièces 6a ou 6b comprend, d'une part, deux parois latérales 10 faisant saillie, en ce qui concerne les éléments d'accrochage 5, du côté opposé à l'organe d'accrochage 5a,5b qu'ils comprennent, ces parois étant sensiblement parallèles et flexibles et délimitant entre elles un canal 11 ajusté au diamètre des tiges 3, et, d'autre part, un élément de serrage 12 apte à être engagé sur les branches latérales 10.

Comme cela apparaît clairement sur les figures, les faces extérieures 10a des parois latérales 10 sont inclinées l'une en direction de l'autre de manière à converger, en ce qui concerne les éléments d'accrochage 5, du côté opposé à l'organe d'accrochage 5a ou 5b et, en ce qui concerne les pièces 6a et 6b, du côté opposé aux vertèbres 4 lorsque le dispositif 2 est mis en place sur le rachis.

Deux fentes 13 sont ménagées dans chaque élément d'accrochage 5 et dans chaque pièce 6a ou 6b, entre les parois latérales 10 et à proximité de leur base.

L'élément de serrage 12 a, quant à lui, une forme d'étrier, dont les branches latérales possèdent des faces intérieures 12a inclinées selon des pentes qui correspondent à celles des faces extérieures 10a des parois latérales 10.

En outre, chaque élément d'accrochage 5 ou pièce 6a,6b comprend deux vis 14 dont les têtes 14a, de forme hexagonale, sont en appui contre la face extérieure de la partie centrale de l'étrier que forme l'élément de serrage 12 et dont les corps 14b traversent les branches parallèles de celui-ci, ces vis 14 étant destinées à être engagées dans des alésages taraudés 15 ménagés dans deux saillies 16 situées à l'extérieur des parois latérales 10 que comprend chaque élément d'accrochage 5 ou chaque pièce 6a,6b.

La figure 4 montre plus particulièrement que la partie centrale des parois latérales 10 peut être usinée, voire évidée, à partir de la face 10a, pour permettre le passage des vis 14 et obtenir ainsi un gain d'encombrement sensible.

En outre, il apparaît sur la figure 4 que les faces internes des parois latérales 10 comprennent, à proximité de leur extrémité supérieure, des bourrelets 20 ou des têtons en regard, la distance séparant lesdits bourrelets 20 ou têtons d'une même paire de parois latérales 10 étant légèrement inférieure au diamètre des tiges 3. Les parois latérales 10 des éléments d'accrochage 5 sont également pourvues de tels bourrelets ou têtons.

En pratique, la mise en forme des tiges 3 selon la ou les courbures du rachis est réglée grâce au clipsage des tiges 3 dans les canaux 11, rendu possible par les bourrelets 20 ou simplement par une distance des faces intérieures des parois latérales 10, au niveau de leur extrémité supérieure, inférieure au diamètre des tiges 3.

Les faces 12a des éléments de serrage 12 sont destinées à venir en appui contre les faces 10a des parois latérales 10 et à les rapprocher l'une de l'autre lorsque les vis 14 sont serrées. Les vis 14 sont faciles à manipuler et leur disposition latérale sur chaque élément d'accrochage 5 ou pièce 6a ou 6b permet de parfaitement contrôler la déformation des parois latérales 10 et, donc, le serrage exercé par elles sur les tiges 3. La précision dans le serrage ainsi obtenue permet dès lors de réaliser un serrage juste suffisant pour immobiliser temporairement les tiges 3 par rapport aux éléments d'accrochage 5, avant blocage définitif, pour une ultime vérification de l'adéquation de leur forme aux courbures du rachis.

De plus et surtout, la fixation des étriers 12 est réalisée au moyen des vis 14, c'est-à-dire de manière indépendante de la flexibilité des parois latérales 10, ce qui permet l'obtention d'une liaison tiges 3/éléments d'ancrage 5 parfaitement solide et résistante dans le temps.

Les éléments transversaux 6, grâce au fait qu'ils sont constitués par deux pièces 6a,6b assemblées l'une à l'autre, peuvent être installés en tout point des tiges 3 et notamment entre deux paires d'organes d' accrochage 5 préalablement mis en place, si cela s'avère nécessaire. Chacune des pièces 6a et 6b est engagée autour de l'une des tiges 3 puis les pièces 6a et 6b sont assemblées l'une à l'autre. Les figures 2 à 4 montrent que la pièce 6a comprend une lumière oblongue 21 ménagée selon son axe longitudinal et que la pièce 6b comprend un alésage taraudé 22, une vis 23 étant prévue pour traverser ladite lumière 21 et être engagée dans ledit alésage 22, puis serrée. La longueur des éléments 6 est ainsi réglable ce qui évite d'avoir à disposer de toute une série d'éléments de longueurs différentes. La pièce 6a comporte en outre une languette 24 tandis que la pièce 6b comprend une rainure 25 correspondante de telle sorte que les pièces 6a,6b puissent être guidées en translation l'une par rapport à l'autre. Toute angulation des tiges 3 est dès lors impossible.

## Revendications

1. Dispositif d'ostéosynthèse vertébrale, comprenant deux tiges rigides (3) de support sur lesquelles peuvent être montés des éléments d'ancrage osseux comprenant des organes d'accrochage (5a,5b) constitués soit par des crochets destinés à être engagés autour des apophyses vertébrales, soit par des vis destinées à prendre appui dans le pédicule des vertèbres, chacun des éléments d'ancrage (5) comprenant d'une part deux parois latérales (10) faisant saillie du côté opposé à son organe d'accrochage, sensiblement parallèles et flexibles, délimitant entre elles un canal (11) ajusté au diamètre des tiges (3) et, d'autre part, un élément de serrage (12) apte à être engagé sur les parois latérales (10) et à les rapprocher l'une de l'autre au cours dudit engagement, caractérisé en ce que la distance séparant les faces intérieures des parois latérales (10), au niveau des extrémités supérieures de celles-ci, est inférieure au diamètre des tiges (3) afin que ces dernières puissent être provisoirement maintenues entre elles par clipsage, et les faces extérieures (10a) des parois latérales (10) de chaque élément d'accrochage (5) sont inclinées l'une en direction de l'autre de manière à converger du côté opposé à l'organe d'accrochage (5a,5b) destiné à être fixé à l'os, tandis que l'élément de serrage (12) a une forme d'étrier, dont les branches latérales possèdent des faces intérieures (12a) inclinées selon des pentes qui correspondent à celles des faces extérieures (10a) des parois latérales (10), au moins deux moyens de serrage (14), disposés au-delà des parois latérales (10), étant prévus pour déplacer ledit élément de serrage (12) de manière à ce que les faces intérieures (12a) de ses branches latérales viennent appuyer contre les parois latérales (10) en les rapprochant l'une de l'autre et glisser sur elles lors du serrage.

2. Dispositif d'ostéosynthèse selon la revendication 1, caractérisé en ce que lesdits moyens de serrage sont constitués par deux vis (14), dont les têtes (14a) sont en appui contre la face extérieure de la partie centrale de l'étrier et dont les corps (14b) traversent les branches parallèles de celui-ci, ces vis (14) étant engagées dans des alésages taraudés (15) ménagés dans deux saillies (16) situées à l'extérieur des parois latérales (10), que comprend chaque élément d'accrochage (5).

3. Dispositif d'ostéosynthèse selon la revendication 2, caractérisé en ce que la partie centrale des parois latérales (10) est usinée, voire évidée, pour permettre le passage des vis (14).

4. Dispositif d'ostéosynthèse selon l'une des revendications 1 à 3, caractérisé en ce que deux lentes (13) sont ménagées dans chaque élément d'accrochage (5), entre les parois latérales (10) et à proximité de leur base.

5. Dispositif d'ostéosynthèse selon la revendication 4, caractérisé en ce que ces lentes (13) sont ménagées radialement par rapport à la tige (3).

6. Dispositif d'ostéosynthèse selon l'une des revendications 1 à 5, caractérisé en ce que les faces internes des parois latérales (10) comprennent, à proximité de leur extrémité supérieure, des bourrelets (20) ou des têtons en regard.

7. Dispositif d'ostéosynthèse selon l'une des revendications 1 à 6, caractérisé en ce qu'il comprend au moins un élément transversal (6) destiné à relier les tiges (3) l'une à l'autre, chacun de ces éléments transversaux étant constitué par deux pièces (6a,6b) pouvant être fixées l'une à l'autre et comportant chacune deux parois latérales (10), un élément de serrage (12) et des moyens de serrage (14) tels que précités.

8. Dispositif d'ostéosynthèse selon la revendication 7, caractérisé en ce que l'une des pièces (6a) de chaque élément transversal (6) comprend une lumière oblongue (21) ménagée selon son axe longitudinal et l'autre pièce (6b) comprend un alésage taraudé (22), une vis (23) étant prévue pour traverser ladite lumière (21) et être engagée dans ledit alésage (22) puis serrée.

9. Dispositif d'ostéosynthèse selon la revendication 8, caractérisé en ce que l'une des pièces (6a) comporte une languette (24) tandis que l'autre pièce (6b) comprend une rainure (25) correspondante.

## Claims

1. Vertebral osteosynthesis device, comprising two rigid support rods (3) on which there can be mounted bone anchoring elements comprising attachment members (5a, 5b) consisting of either hooks designed to be engaged around vertebral apophyses, or screws designed to bear in the pedicle of the vertebrae, each of the anchoring elements (5) comprising on the one hand two lateral walls (10) projecting on the side opposite to its attachment member, substantially parallel and flexible, defining between them a channel (11) adjusted to the diameter of the rods (3) and, on the other hand, a clamping element (12) suitable for being engaged on the lateral walls (10) and for bringing them closer together during the said engagement, characterised in that the distance separating the internal faces of the lateral walls (10), at the top ends thereof, is less than the diameter of the rods (3) so that the latter can be held temporarily between them by snapping in, and the external faces (10a) of the lateral walls (10) of each attachment element (5) are inclined towards to each other so as to converge on the side opposite to the attachment member (5a, 5b) designed to be fixed to the bone, whilst the clamping element (12) is in the shape of a stirrup, the lateral legs of which have internal faces (12a) inclined with slopes which correspond to those of the external faces (10a) of the lateral walls (10), at least two clamping means (14), disposed beyond the lateral walls (10), being provided to move the said clamping element (12) so that the internal faces (12a) of its lateral legs come to bear against the lateral walls (10) whilst moving them closer together and slide on them during clamping.

2. Osteosynthesis device according to Claim 1, characterised in that the said clamping means consist of two screws (14), the heads (14a) of which are in abutment against the external face of the central part of the stirrup and the bodies (14b) of which pass through the parallel legs thereof, these screws (14) being engaged in, threaded bores (15) formed in two projections (16) situated outside the lateral walls (10), which each attachment element (5) comprises.

3. Osteosynthesis device according to Claim 2, characterised in that the central part of the lateral walls (10) is machined, or even hollowed out, to allow passage of the screws (14).

4. Osteosynthesis device according to one of Claims 1 to 3, characterised in that two slots (13) are formed in each attachment element (5), between the lateral walls (10) and close to their base.

5. Osteosynthesis device according to Claim 4, characterised in that these slots (13) are formed radially with respect to the rod (3).

6. Osteosynthesis device according to one of Claims 1 to 5, characterised in that the internal faces of the lateral walls (10) comprise, close to their top end, protrusions (20) or studs facing each other.

7. Osteosynthesis device according to one of Claims 1 to 6, characterised in that it comprises at least one transverse element (6) designed to connect the rods (3) to each other, each of these transverse elements consisting of two parts (6a, 6b) which can be fixed to each other and each having two lateral walls (10), a clamping element (12) and clamping means (14) as mentioned above.

8. Osteosynthesis device according to Claim 7, characterised in that one of the parts (6a) of each transverse element (6) comprises an oblong aperture (21) formed along its longitudinal axis and the other part (6b) comprises a threaded bore (22), a screw (23) being provided to pass through the said aperture (21) and be engaged in the said bore (22) and then tightened.

9. Osteosynthesis device according to Claim 8, characterised in that one of the parts (6a) has a tongue (24) whilst the other part (6b) comprises a corresponding groove (25).

## Patentansprüche

1. Osteosynthesevorrichtung für die Wirbelsäule mit zwei starren Haltestangen (3), auf denen Elemente zur Knochenverankerung montiert werden können, welche Aufhängungseinrichtungen (5a, 5b) aufweisen, die entweder aus Haken, welche die Wirbelfortsätze umgreifen können, oder aus Schrauben, welche gegen das Wirbelpedikel drücken können, bestehen, wobei jedes der Verankerungselemente (5) einerseits zwei im wesentlichen parallele und biegsame Seitenwände (10) aufweist, die auf der ihrer Aufhängungseinrichtung gegenüberliegenden Seite überstehen und dazwischen einen aufden Durchmesser der Stangen (3) eingestellten Kanal (11) begrenzen, und andererseits ein Klemmelement (12) aufweist, das auf den Seitenwänden (10) zum Eingriff gebracht werden kann und sie während dieses Eingriffs einander annähern kann, **dadurch gekennzeichnet,** daß die zwischen den Innenflächen der Seitenwände (10) liegende Entfernung auf der Höhe der oberen Enden kleiner als der Durchmesser der Stangen (3) ist, so daß letztere provisorisch durch Aufklipsen zwischen ihnen gehalten werden können, und daß die Außenflächen (10a) der Seitenwände (10) jedes Aufhängungselements (5) aufeinander zu geneigt sind, so daß sie auf der gegenüberliegenden Seite der zur Befestigung am Knochen dienenden Aufhängungseinrichtung (5a, 5b) konvergieren, während das Klemmelement (12) die Form eines Bügels hat, dessen Seitenschenkel Innenflächen (12a) besitzen, welche Neigungen aufweisen, die denjenigen der Außenflächen (10a) der Seitenwände (10) entsprechen, wobei mindestens zwei jenseits der Seitenwände (10) angeordnete Klemmeinrichtungen (14) vorgesehen sind, um das Klemmelement (12) derart zu verschieben, daß die Innenflächen (12a) seiner Seitenschenkel gegen die Seitenwände (10) drücken, wobei sie einander angenähert werden, und auf ihnen während des Festklemmens gleiten.

2. Osteosynthesevorrichtung nach Anspruch 1, dadurch gekennzeichnet daß die Klemmeinrichtungen aus zwei Schrauben (14) bestehen, deren Köpfe (14a) gegen die Außenfläche des mittigen Teils des Bügels drücken und deren Körper (14b) dessen parallele Schenkel durchqueren, wobei die Schrauben (14) in Bohrungen mit Innengewinde (15) eingreifen, welche in zwei Vorsprünge (16) eingearbeitet sind, die sich außen an den Seitenwänden (10) befinden, die jedes Aufhängungselement (5) aufweist.

3. Osteosynthesevorrichtung nach Anspruch 2, dadurch gekennzeichnet, daß der mittige Teil der Seitenwände (10) maschinell bearbeitet und sogar mit einer Aussparung versehen ist, um den Durchtritt der Schrauben (14) zu ermöglichen.

4. Osteosynthesevorrichtung nach einem der Ansprüche 1 bis 3, dadurch gekennzeichnet, daß zwischen den Seitenwänden (10) und in der Nähe ihrer Basis zwei Schlitze (13) in jedes Aufhängungselement (5) eingearbeitet sind.

5. Osteosynthesevorrichtung nach Anspruch 4, dadurch gekennzeichnet, daß die Schlitze (13) bezüglich der Stange (3) radial eingearbeitet sind.

6. Osteosynthesevorrichtung nach einem der Ansprüche 1 bis 5, dadurch gekennzeichnet, daß die Innenflächen der Seitenwände (10) in der Nähe ihres oberen Endes gegenüberliegende Wülste (20) oder Ansätze aufweisen.

7. Osteosynthesevorrichtung nach einem der Ansprüche 1 bis 6, dadurch gekennzeichnet, daß sie zumindest ein Querelement (6) aufweist, um die Stangen (3) miteinander zu verbinden, wobei jedes dieser Querelemente aus zwei Stücken (6a, 6b) besteht, die aneinander befestigt werden können, und jedes von ihnen zwei Seitenwände (10), ein Klemmelement (12) und Klemmeinrichtungen (14), wie zuvor genannt, aufweist.

8. Osteosynthesevorrichtung nach Anspruch 7, dadurch gekennzeichnet, daß das eine der Stücke (6a) jedes Querelements (6) ein entlang seiner Längsachse eingearbeitetes Längsloch (21) aufweist, und das andere Stück (6b) eine Bohrung mit Innengewinde (22) aufweist, wobei eine Schraube (23) zur Durchquerung des Längslochs (21) und zum Eingriff in die Bohrung (22) vorgesehen ist, die dann angezogen wird.

9. Osteosynthesevorrichtung nach Anspruch 8, dadurch gekennzeichnet, daß eines der Stücke (6a) eine Zunge (24) aufweist, während das andere Stück (6b) eine entsprechende Rille (25) aufweist.
